# EUROPÄISCHE PATENTSCHRIFT

(11) **EP 1 316 294 B1**
(45) Veröffentlichungstag und Bekanntmachung des Hinweises auf die Patenterteilung: **09.01.2008**
(21) Anmeldenummer: 02025336.5
(22) Anmeldetag: 13.11.2002
(51) Int. Cl.: A61B 17/70

(54) **Verschlusseinrichtung zum Sichern eines stabförmigen Elements in einem mit einem Schaft verbundenen Halteelement**
Fastener assembly for securing a rod-shaped element in a retaining element connected to a shaft
Dispositif de fixation pour sécuriser un élément en forme de barre dans un élément de retenue relié à une tige

(30) Priorität: 27.11.2001 DE 10157814
(43) Veröffentlichungstag der Anmeldung: 04.06.2003
(73) Patentinhaber: BIEDERMANN MOTECH GmbH, 78054 VS-Schwenningen (DE)
(72) Erfinder: Biedermann, Lutz, 78048 VS-Villingen (DE)
(74) Vertreter: Hofer, Dorothea

(56) Entgegenhaltungen:
- EP-A- 0 280 748
- WO-A-95/01132
- DE-U- 29 810 798
- GB-A- 258 106
- US-A- 6 063 090
- US-A- 6 074 391

## Beschreibung

Die Erfindung betrifft eine in der Wirbelsäulen- bzw. Unfallchirurgie zu verwendende Verschlußeinrichtung zum Sichern eines stabförmigen Elements in einem mit einem Schaft verbundenen Halteelement. Ferner betrifft die Erfindung ein Element mit einem Schaft und einem damit verbundenen Halteelement zum Verbinden mit einem Stab mit einer derartigen Verschlußeinrichtung.

Aus der WO 95/01132 und der US 6,224,598 B1 ist jeweils ein Wirbelsäulenimplantat mit einem Aufnahmeteil mit einer U-förmigen Ausnehmung zur Aufnahme eines Stabes bekannt, wobei die durch die U-förmige Ausnehmung gebildeten freien Schenkel ein Innengewinde aufweisen. Zur Fixierung des Stabes ist ein zwischen die Schenkel einschraubbares Verschlußelement mit einem Außengewinde, einer zentralen Bohrung mit Innengewinde und eine in das Verschlußelement einschraubbare Innenschraube vorgesehen. Bei der aus der WO 95/01132 bekannten Vorrichtung wird zum Vermeiden des Aufspreizens der Schenkel eine die Schenkel von außen umfassende Sicherheitshülse verwendet.

Aufgabe der Erfindung ist es, eine Verschlußeinrichtung der eingangs beschriebenen Art zu schaffen, welche eine verbesserte Sicherung gegen Lockerung oder Lösen der Verschlußelemente gewährleistet und gleichzeitig kompakt gebaut und einfach herzustellen ist.

Diese Aufgabe wird durch die in Patentanspruch 1 gekennzeichnete Verschlußeinrichtung bzw. durch das in Patentanspruch 5 gekennzeichnete Element gelöst. Weiterbildungen der Erfindung sind in den Unteransprüchen gegeben.

Die Erfindung ist sowohl für monoaxiale als auch für polyaxiale Knochenschrauben geeignet, da sie es erlaubt, daß die Fixierung des Stabes bzw. des Stabes und des Kopfes der Knochenschraube im endgültigen Zustand so zusammenwirken, daß ein sicheres Verblocken gegen Lockern oder Lösen des Verschlußmechanismus besteht.

Weitere Merkmale und Zweckmäßigkeiten der Erfindung ergeben sich aus der Beschreibung von Ausführungsbeispielen anhand der Figuren. Von den Figuren zeigen:
- Fig. 1:: eine erste Ausführungsform in perspektivischer Explosionsdarstellung;
- Fig. 2a:: eine schematische Darstellung der auf das Verschlußelement von Fig. 1 wirkenden Kräfte vor dem Einschrauben der Innenschraube;
- Fig. 2b:: eine schematische Darstellung der auf das Verschlußelement von Fig. 1 wirkenden Kräfte nach dem Einschrauben der Innenschraube;
- Fig. 3:: eine Schnittansicht einer zweiten Ausführungsform;
- Fig. 4:: eine Schnittansicht einer dritten Ausführungsform;
- Fig. 5:: eine Schnittansicht eines Details einer vierten Ausführungsform;
- Fig. 6:: eine Schnittansicht eines Details einer fünften Ausführungsform;
- Fig. 7:: eine Schnittansicht eines Details einer sechsten Ausführungsform; und
- Fig. 8:: eine Schnittansicht eines Details einer siebten Ausführungsform.

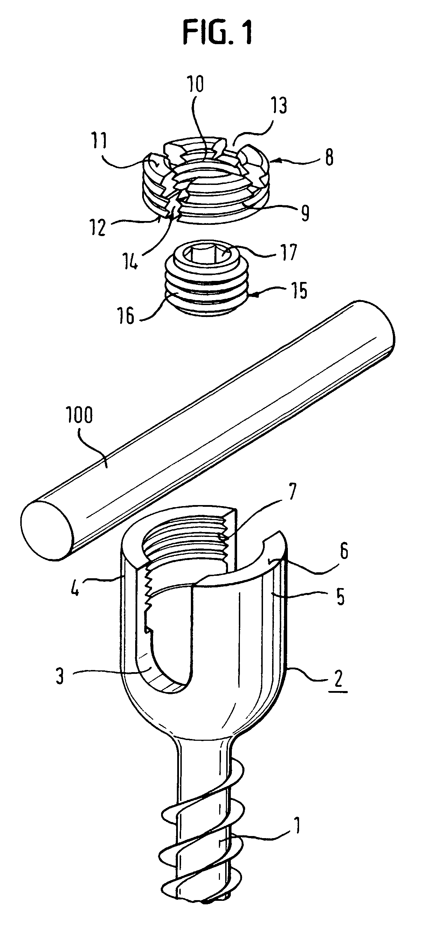

Das erfindungsgemäße Element ist in der in der in den Figuren Fig. 1 dargestellten Ausführungsform als Monoaxial-Knochenschraube ausgebildet. Diese weist einen Schaft 1 mit einem Knochengewindeabschnitt und ein damit starr verbundenes Aufnahmeteil 2 zur Aufnahme eines die Knochenschraube mit weiteren Knochenschrauben verbindenden Stabes 100 auf. Hierzu ist das Aufnahmeteil mit einer Ausnehmung 3 mit einem U-förmigen Querschnitt versehen, die gerade so groß bemessen ist, daß der Stab 100 einlegbar ist und in den Grund der Ausnehmung passt. Durch die U-förmige Ausnehmung 3 sind zwei freie Schenkel 4, 5 mit jeweils einem freien Ende 6 gebildet, welches den oberen Rand des Aufnahmeteils 2 bildet. Angrenzend an das freie Ende 6 weisen die Schenkel 4, 5 ein Innengewinde 7 auf.

Zum Verschließen des Stabes 100 in der Ausnehmung 3 ist ein zwischen die Schenkel 4, 5 einschraubbares muffen- bzw. mutterartiges Verschlußelement 8 mit einem mit dem Innengewinde 7 der Schenkel zusammenwirkenden Außengewinde 9 und einem Innengewinde 10 vorgesehen. Das Verschlußelement 8 weist ein erstes stirnseitiges Ende 11 und diesem gegenüberliegend ein zweites Ende 12 auf. Zum Ineingriffbringen mit einem Einschraubwerkzeug sind an dem ersten stirnseitigen Ende 11 radial verlaufende Einschnitte 13 vorgesehen. Einer der Einschnitte erstreckt sich durch das gesamte Verschlußelement 8 in axialer Richtung und bildet somit einen Schlitz 14 derart, daß das Verschlußelement nach Art eines Spannrings eine gewisse Elastizität aufweist. Die Länge des Verschlußelements 8 in axialer Richtung ist derart bemessen, daß das Verschlußelement vollständig zwischen die Schenkel 4, 5 einschraubbar ist.

Ferner ist eine in das Verschlußelement einschraubbare Innenschraube bzw. Klemm- oder Setzschraube 15 vorgesehen, deren Außengewinde 16 mit dem Innengewinde 10 des Verschlußelements 8 zusammenwirkt. Die Innenschraube 15 weist an ihrem einen Ende eine Ausnehmung 17 zum Ineingriffbringen mit einem Schraubwerkzeug auf.

Bei der in Fig. 1 gezeigten Ausführungsform sind das Innengewinde 7 der Schenkel, daß Außengewinde 9 des Verschlußelements, sowie das Innengewinde 10 des Verschlußelements und das Außengewinde 16 der Innenschraube 15 als metrische Gewinde ausgebildet.

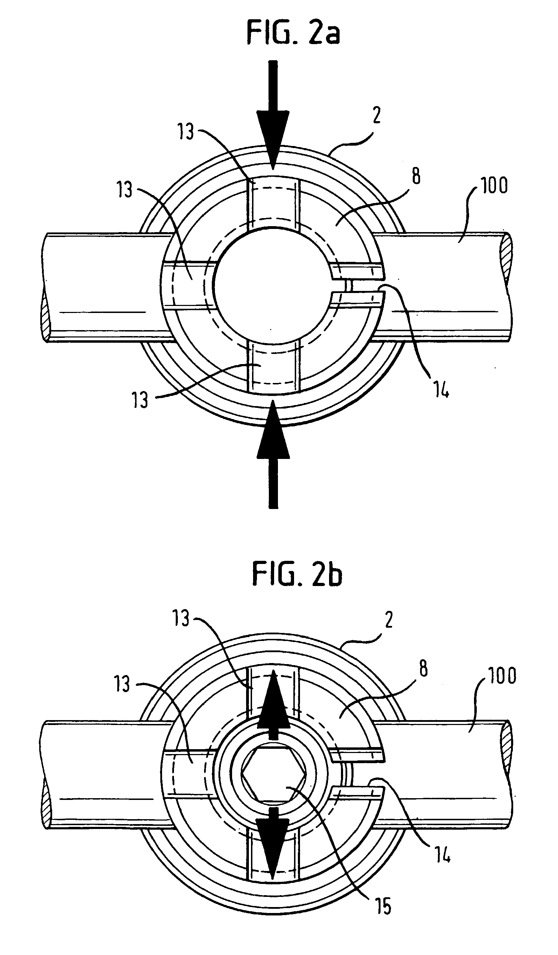

Im Betrieb wird die Knochenschraube zuerst in den Knochen eingeschraubt und anschließend der Stab 100 eingelegt. Dann wird das Verschlußelement 8 mit bereits eingeschraubter, aber noch nicht mit ihrer Unterseite den Stab berührender Innenschraube 15 eingeschraubt, wodurch der Stab zunächst nur am Herauskippen gehindert wird, aber noch verschiebbar ist. Beim Einschrauben des Verschlußelements 8 kommt es aufgrund der geschlitzten und damit elastischen Ausbildung desselben nicht zu einem Aufspreizen der Schenkel 4, 5, sondern, wie in Fig. 2a gezeigt ist, setzen die Schenkel dem geschlitzten Verschlußelement einen Widerstand entgegen, der es versucht zusammenzudrücken, was durch die nach innen gerichteten Pfeile veranschaulicht ist. Anschließend wird die Innenschraube 15 so eingeschraubt bis sie mit ihrer Unterseite auf den Stab drückt und diesen fixiert. Durch das Festziehen der Innenschraube 15 wirkt auf das Verschlußelement 8 wie in Fig. 2b durch die Pfeile dargestellt ist, eine nach radial nach außen gerichtete Kraftkomponente, wodurch das Außengewinde 9 des Verschlußelements in das Innengewinde 7 der Schenkel gedrückt wird und sich dort verblockt. Damit ist ein gegen Lockerung gesicherter Verschluß gewährleistet.

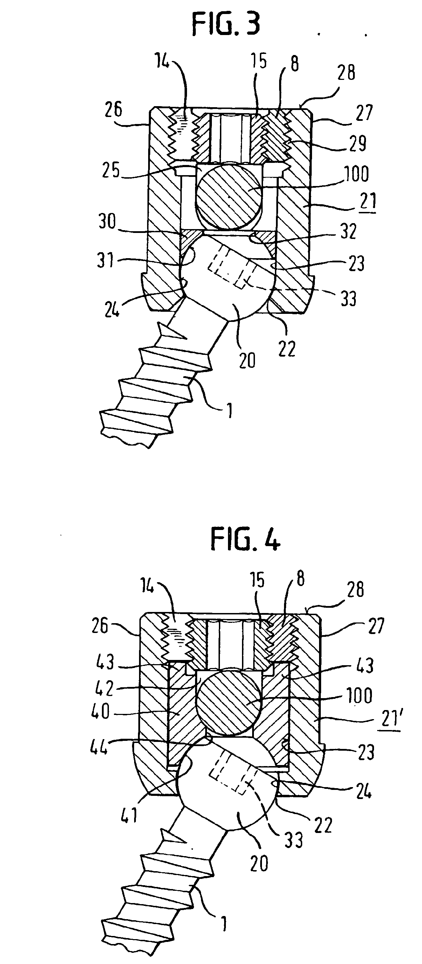

In der in Fig. 3 gezeigten Ausführungsform ist das erfindungsgemäße Element als Polyaxialschraube ausgebildet, die ein Schraubenelement mit einem Gewindeschaft 1 mit einem Knochengewinde und einem kugelsegmentförmigen Kopf 20 aufweist, der mit einem Aufnahmeteil 21 verbunden ist Das Aufnahmeteil 21 hat an seinem einen Ende eine axialsymmetrisch ausgerichtete erste Bohrung 22, deren Durchmesser größer als der des Gewindeabschnitts des Schafts 1 und kleiner als der des Kopfs 20 ist. Ferner weist das Aufnahmeteil 21 eine koaxiale zweite Bohrung 23 auf, die auf dem der ersten Bohrung 22 gegenüberliegenden Ende offen ist und deren Durchmesser so groß ist, daß das Schraubenelement durch das offene Ende mit seinem Gewindeabschnitt durch die erste Bohrung 22 hindurch und mit dem Kopf 20 bis zum Grund der zweiten Bohrung 23 führbar ist. Zwischen der ersten und der zweiten Bohrung ist ein kleiner koaxialer Abschnitt 24 vorgesehen, der unmittelbar an die erste Bohrung 22 angrenzt und zum offenen Ende hin sphärisch ausgebildet ist, wobei der Radius im wesentlichen gleich dem kugelsegmentförmigen Abschnitts des Kopfs 20 ist. Das Aufnahmeteil 21 weist ebenso wie das Aufnahmeteil 2 der ersten Ausführungsform eine zur Mitte des Teiles symmetrisch angeordnete U-förmige Ausnehmung 25 auf, deren Grund zur ersten Bohrung 22 hin gerichtet ist und durch die zwei freie Schenkel 26, 27 gebildet sind, deren freies Ende 28 den oberen Rand des Aufnahmeteils 21 bilden. In einem Bereich angrenzend an das freie Ende 28 weisen die Schenkel 26, 27 ein Innengewinde 29 auf.

Es ist ferner ein Druckelement 30 vorgesehen, das so ausgebildet ist, daß es an seiner dem Kopf 20 zugewandten Seite eine sphärische Ansenkung 31 aufweist, deren Radius im wesentlichen gleich dem Radius des kugelsegmentförmigen Abschnitts des Kopfes 20 ist. Der Außendurchmesser des Druckelements ist so gewählt, daß das Druckelement in dem Aufnahmeteil 21 eine Gleitbewegung durchführen kann, also zu dem Kopf 20 hin verschiebbar ist. Das Druckelement weist ferner eine koaxiale Bohrung 32 für den Zugriff auf eine Ausnehmung 33 in dem Schraubenkopf 20 zum Ineingriffbringen mit einem Eindrehwerkzeug auf.

Die Ausbildung des Verschlußelements 8 und der Innenschraube 15 ist wie bei der ersten Ausführungsform.
Im Betrieb wird das Schraubenelement nach Einsetzen in das Aufnahmeteil 21 in den Knochen eingeschraubt. Dann werden nacheinander das Druckelement 30 und der Stab 100 eingesetzt. In diesem Stadium ist der Schraubenkopf 20 noch verschwenkbar. Durch Einschrauben des Verschlußelements 8 und der Innenschraube 15 in derselben Weise wie bei der ersten Ausführungsform werden das Schraubenelement und das Aufnahmeteil 21 zueinander und damit auch der Stab 100 fixiert.

Die in Fig. 4 dargestellte dritte Ausführungsform zeigt ebenfalls eine Polyaxial-Knochenschraube. Der zweiten Ausführungsform entsprechende Teile sind mit denselben Bezugszeichen versehen. Die dritte Ausführungsform unterscheidet sich von der zweiten Ausführungsform im wesentlichen durch die Ausbildung des Druckelements 40.

Das Druckelement 40 dieser Ausführungsform ist im wesentlichen zylindrisch ausgebildet mit einem Außendurchmesser, der so gewählt ist, dass das Druckelement 40 in der zweiten Bohrung 23 des Aufnahmeteils 21' gleiten kann. An seinem einen Ende ist eine sich zu dem Ende hin erweiternde kugelsegmentförmige Ausnehmung 41 vorgesehen, deren Kugelradius so gewählt ist, dass er in einem in das Aufnahmeteil eingesetzten Zustand den Kopf 20 des Schraubenelements teilweise umfasst. Ein wesentlicher Unterschied zu dem Druckelement 30 der zweiten Ausführungsform besteht darin , daß das Druckelement40 der dritten Ausführungsform in Richtung des freien Endes 28 der Schenkel verlängert ist. Dazu weist es an seinem der kugelsegmentförmigen Ausnehmung 41 gegenüberliegenden Ende eine U-förmige Ausnehmung 42 auf, wobei die Abmessungen der U-förmigen Ausnehmungen des Druckelements so bemessen sind, dass in den dadurch gebildeten Kanal der Stab 100 eingelegt werden kann. Die in Richtung der Zylinderachse des Aufnahmeteils 21' gesehene Tiefe der U-förmigen Ausnehmung 42 ist größer als der Durchmesser des aufzunehmenden Stabs 100, so dass das Druckelement 40 mit seitlichen Schenkeln 43 über den eingelegten Stab 100 nach oben hervorsteht. Das Druckelement 40 weist ferner eine zentrale Bohrung 44 auf, die sich durch dieses hindurch erstreckt. Der Durchmesser der zentralen Bohrung 44 ist gerade so groß bemessen, dass ein Schraubwerkzeug zum Ineingriffbringen mit der in dem Kopf 20 vorgesehenen Ausnehmung 33 hindurchführbar ist.

Die Ausbildung des Verschlußelements 8 und der Innenschraube 15 ist wie bei der ersten und zweiten Ausführungsform.

Im Betrieb unterscheidet sich die in Fig. 4 gezeigte dritte Ausführungsform von der in Fig. 3 gezeigten zweiten Ausführungsform darin, daß über das Verschlußelement 8 zuerst der Kopf 20 in seiner Stellung blockiert wird, in dem das Verschlußelement 8 auf die verlängerten Schenkel 43 des Druckelements 40 drückt und damit auf den Kopf 20. Durch Festziehen der Innenschraube 15 wird dann der Stab 100 unabhängig von dem Kopf 20 fixiert. Die Verschließwirkung durch das Zusammenwirken von Verschlußelement 8 und Innenschraube 15 ist im übrigen wie bei der ersten und zweiten Ausführungsform.

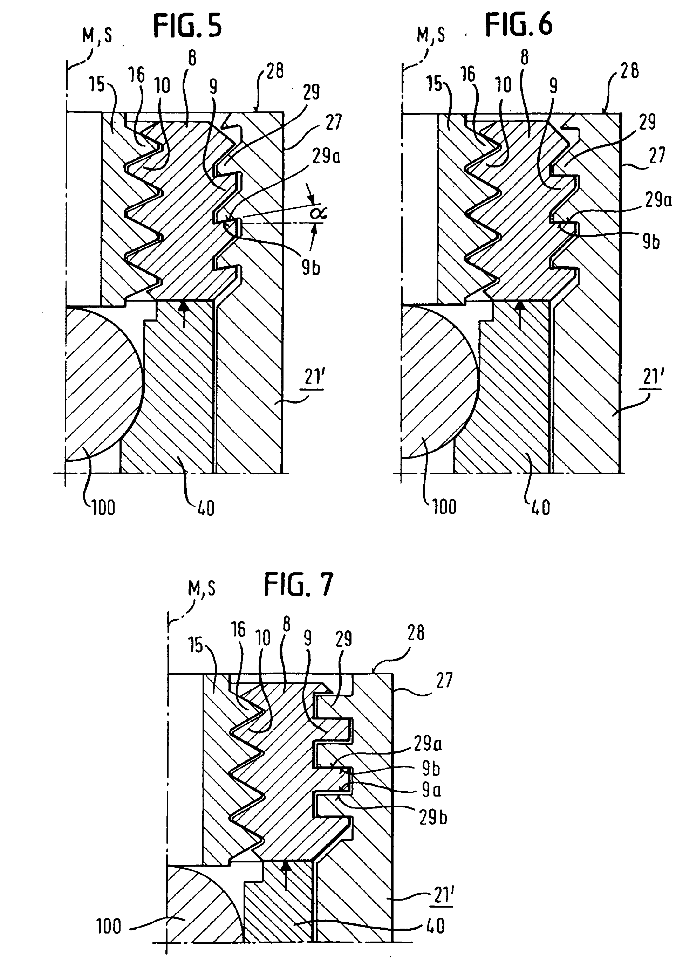

Die in den Figuren 5 bis 7 gezeigten Ausführungsformen unterscheiden sich von der in Fig. 4 gezeigten dritten Ausführungsform durch die Art des Innengewindes 7 der Schenkel 26, 27 des Aufnahmeteils 21' und das entsprechende Außengewinde 9 des Verschlußelements 8. Das Innengewinde 10 des Verschlußelements 8 und das Außengewinde 16 der Innenschraube sind bei diesen Ausführungsformen jeweils als metrische Gewinde ausgebildet.

In der in Fig. 5 gezeigten Ausführungsform ist das Innengewinde 29 der Schenkel 26, 27 so ausgebildet, daß die dem freien Ende abgewandte Flanke 29a mit einer zur Mittenachse M des Aufnahmeteils senkrecht verlaufenden Ebene einen negativen Winkel α einschließt. Entsprechend schließt die in eingeschraubtem Zustand dem freien Ende abgewandte Flanke 9b des Außengewindes 9 des Verschlußelements 8 mit einer zur Schraubenachse S horizontal verlaufenden Ebene einen negativen Winkel ein. Die Maße von Innengewinde der Schenkel und Außengewinde des Verschlußelements sind derart, daß in unbelastetem Zustand zwischen den genannten Flanken 29a und 19b ein Zwischenraum ist.

Im Betrieb erfahren die Schenkel 26, 27 durch den negativen Flankenwinkel beim Festziehen des Verschlußelements 8 wobei, daß die Flanken 29a und 10b aufeinandergelangen eine radial nach innen gerichtete Kraftkomponente, so daß die Schenkel etwas zusammengezogen werden. Damit erfolgt eine zuverlässige Blockierung des Kopfes 20 über das Druckelement 40. Beim Einschrauben der Innenschraube spreizt sich das Verschlußelement 8 aufgrund der dann radial nach außen wirkenden Kraftkomponente und wirkt somit der Kraft, die die Schenkel zusammenzieht entgegen. Der Stab wird fixiert und gleichzeitig erfolgt ein gegeneinander Blockieren von Verschlußelelement 8 und Schenkel.

Die in Fig. 6 gezeigte Ausführungsform unterscheidet sich von der in Fig. 5 gezeigten Ausführungsform durch die Ausbildung der Gewinde 29, 9 als Sägengewinde mit einer horizontal zur Mittenachse M bzw. Schraubenachse S verlaufenden Lastflanke 29a bzw. 19b. Auch damit ist die erfindungsgemäße Funktion des Verschlußelements gegeben.

Die in Fig. 7 gezeigte Ausführungsform unterscheidet sich von der in Fig. 5 bzw. 6 gezeigten Ausführungsform dadurch, daß das Innengewinde 29 der Schenkel und das Außengewinde 9 des Verschlußelements als Flachgewinde ausgebildet sind. Dieses weist zwei horizontal zur Mittenachse M bzw. Schraubenachse S verlaufende Flanken 29a, 29b bzw. 9a, 9b auf. Der Gewindequerschnitt ist im wesentlichen rechteckig ausgebildet. Auch damit ist die erfindungsgemäße Funktion des Verschlußelements gegeben.

Auch bei den in Fig. 6 und 7 gezeigten Gewindeformen ist ein Flankenspiel derart vorgesehen, daß das Verblocken des Verschlußelements nach Einschrauben der Innenschraube möglich ist.

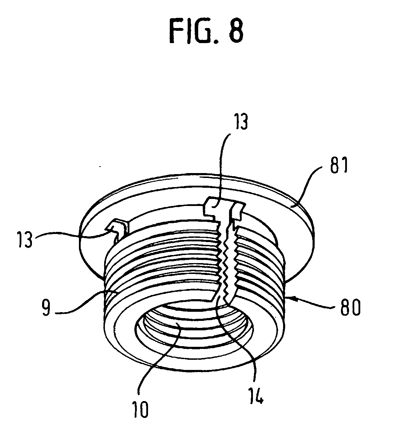

In der in Fig. 8 gezeigten Ausführungsform ist das Verschlußelement 80 mit einem ringförmigen Ansatz 81 an seinem stirnseitigen, die Einschnitte 13 aufweisenden Ende versehen. Der Schlitz 14 erstreckt sich in diesem Fall wie bei dem Verschlußelement 8 wenigstens durch den axialen Abschnitt, in dem das Außengewinde 9 vorgesehen ist, jedoch nicht durch den Ansatz 81. Der Schlitz 14 ist ferner in Umfangsrichtung so angeordnet, daß er sich in einen der Einschnitte 13 erstreckt.

Im Betrieb bildet der Ansatz 81 beim Einschrauben des Verschlußelements einen Anschlag, der die Kraft auf das Druckelement auf einen vorbestimmten Wert begrenzt.

Weitere Ausführungsformen der Erfindung ergeben sich durch das Vorsehen der Gewindearten gemäß den Ausführungsformen nach Fig. 5 bis 6 und/oder des Verschlußelements nach Fig. 8 bei einer Monoaxialschraube gemäß der ersten Ausführungsform nach Fig. 1 oder der Polyaxialschraube gemäß der zweiten Ausführungsform nach Fig. 3.

Anstelle der oben beschriebenen Ausführungen, bei denen der das Aufnahmeteil mit einer Knochenschraube verbunden ist, kann das Aufnahmeteil auch mit einem Haken verbunden sein, wie er in der Wirbelsäulenchirurgie zum Einhaken hinter Knochenvorsprüngen der Wirbelsäule verwendet wird.

In einer weiteren Abwandlung der polyaxialen Ausführungsformen ist anstelle des Gewindeschaftes 1 oder des Hakens eine Stange oder ein stabförmiges Element vorgesehen, das an beiden Enden einen kugelsegmentförmigen Kopf hat, der mit einem Aufnahmeteil der beschriebenen Art verbunden ist. Damit kann ein solches Element als Verbindungselement zwischen zwei Stäben 100 verwendet werden.

## Patentansprüche

1. Verschlußeinrichtung zum Sichern eines stabförmigen Elements in einem mit einem Schaft (1) verbundenen Halteelement (2; 21; 21') zum Einsatz in der Wirbelsäulen- oder Unfallchirurgie, mit einem Verschlußelement (8; 80) mit einer im wesentlichen hohlzylindrischen Form mit einer Rotationsachse, einem Außengewinde (9) und einem Innengewinde (10)
**gekennzeichnet durch** einen sich in axialer Richtung **durch** das Verschlußelement (8) erstreckenden Schlitz (14).

2. Verschlußeinrichtung nach Anspruch 1, **gekennzeichnet durch** eine in das Verschlußelement (8; 80) einschraubbare Innenschraube (15).

3. Verschlußeinrichtung nach Anspruch 1 oder 2, **dadurch gekennzeichnet, daß** das Innengewinde (10) als metrisches Gewinde ausgebildet ist.

4. Verschlußeeinrichtung nach einem der Ansprüche 1 bis 3,
**dadurch gekennzeichnet, daß** das Außengewinde (9) als metrisches Gewinde oder als Gewinde mit horizontalem oder negativem Winkel der Lastflanke oder als Flachgewinde mit zwei horizontalen Flanken ausgebildet ist.

5. Element mit einem Schaft (1) und einem damit verbundenen Halteelement (2; 21; 21') zum Verbinden mit einem Stab (100), wobei das Halteelement (2; 21; 21') eine einen U-förmigen Querschnitt aufweisende Ausnehmung zur Aufnahme des Stabes mit zwei an einem Ende (6; 28) freien Schenkeln (4, 5; 26, 27) und einem Innengewinde (7; 29) an den freien Schenkeln aufweist und mit einem Verschlußelement (8; 80) mit einem Außengewinde (9) in wenigstens einem Abschnitt seiner Außenwand, wobei das Außengewinde (9) mit dem Innengewinde (7; 29) der Schenkel zusammenwirkt und wobei das Verschlußelement eine zentrale Bohrung aufweist,
**dadurch gekennzeichnet, daß** das Verschlußelement (8, 80) einen sich im wesentlichen in axialer Richtung durch seine Außenwand erstreckenden Schlitz (14) aufweist.

6. Element nach Anspruch 5, **dadurch gekennzeichnet, daß** das Verschlußelement (8, 80) ein Innengewinde (10) aufweist und eine Innenschraube (15) zum Einschrauben in das Verschlußelement vorgesehen ist.

7. Element nach Anspruch 5 oder 6, **dadurch gekennzeichnet, daß** das Innengewinde (10) des Verschlußelements (8; 80) und das damit zusammenwirkende Außengewinde (16) der Innenschraube (15) als metrische Gewinde ausgebildet sind.

8. Element nach einem der Ansprüche 5 bis 7, **dadurch gekennzeichnet, daß** das Innengewinde (7) der Schenkel (4, 5; 26, 27) als metrisches Gewinde ausgebildet sind.

9. Element nach einem der Ansprüche 5 bis 7, **dadurch gekennzeichnet, daß** das Innengewinde (7) der Schenkel (4, 5; 26,a b) als Gewinde mit einem negativen Flankenwinkel der Lastflanke ausgebildet ist.

10. Element nach einem der Ansprüche 5 bis 7, **dadurch gekennzeichnet, daß** das Innengewinde (7) der Schenkel (4, 5; 26, 27) als Sägengewinde mit einem im wesentlichen horizontalen Winkel der Lastflanke (29a) ausgebildet ist.

11. Element nach einem der Ansprüche 5 bis 7, **dadurch gekennzeichnet, daß** das Innengewinde (7) der Schenkel (4, 5; 26, 27) als Flachgewinde zwei horizontale Flanken (29a, 29b) ausgebildet ist.

12. Element nach einem der Ansprüche 5 bis 11, **dadurch gekennzeichnet, daß** das Außengewinde (9) des Verschlußelements (8, 80) mit einem zu dem Innengewinde (7; 29) der Schenkel (4, 5; 26, 27) passenden Gewindetyp ausgebildet ist.

13. Element nach einem der Ansprüche 5 bis 12, **dadurch gekennzeichnet, daß** das Verschlußelement (80) einen Anschlag (81) aufweist.

14. Element nach einem der Ansprüche 5 bis 13, **dadurch gekennzeichnet, daß** der Schaft (1) und das Halteelement (2) monoaxial verbunden sind.

15. Element nach einem der Ansprüche 5 bis 13, **dadurch gekennzeichnet, daß** der Schaft (1) und das Halteelement (21; 21') polyaxial verbunden sind.

16. Element nach Anspruch 15, **dadurch gekennzeichnet, daß** ein in dem Halteelement (21') vorgesehenes Druckelement (40) vorgesehen ist, auf welches das Verschlußelement (8, 80) zum Fixieren der Winkelstellung des Schafts (1) einwirkt.

17. Element nach Anspruch 16, **dadurch gekennzeichnet daß** die Winkelstellung des Schafts über das Verschlußelement (8, 80) und der Stab über die Innenschraube (15) fixierbar sind.

## Claims

1. Fastener assembly for securing a rod-shaped element in a retaining element (2; 21; 21') connected to a shaft (1), for use in spinal surgery or accident and emergency surgery, with a fastener element (8; 80) of substantially hollow cylindrical shape with a rotation axis, an outer thread (9) and an inner thread (10), **characterized by** a slit (14) extending in the axial direction through the fastener element (8).

2. Fastener assembly according to Claim 1, **characterized by** an inner screw (15) that can be screwed into the fastener element (8; 80).

3. Fastener assembly according to Claim 1 or 2, **characterized in that** the inner thread (10) is designed as a metric thread.

4. Fastener assembly according to one of Claims 1 to 3, **characterized in that** the outer thread (9) is designed as a metric thread or as a thread with a horizontal or negative angle of the load-bearing flank or as a flat thread with two horizontal flanks.

5. Element with a shaft (1), and with a retaining element (2; 21; 21') which is connected to the latter and which serves for connection to a rod (100), the retaining element (2; 21; 21') having a recess with a U-shaped cross section for receiving the rod and with two free legs (4, 5; 26, 27) at one end (6; 28) and an inner thread (7; 29) on the free legs, and with a fastener element (8; 80) which has an inner thread (9) in at least one portion of its outer wall, the outer thread (9) cooperating with the inner thread (7; 29) of the legs, and the fastener element having a central bore, **characterized in that** the fastener element (8, 80) has a slit (14) extending substantially in an axial direction through its outer wall.

6. Element according to Claim 5, **characterized in that** the fastener element (8, 80) has an inner thread (10), and an inner screw (15) for screwing into the fastener element is provided.

7. Element according to Claim 5 or 6, **characterized in that** the inner thread (10) of the fastener element (8; 80) and the outer thread (16) of the inner screw (15) cooperating therewith are designed as metric threads.

8. Element according to one of Claims 5 to 7, **characterized in that** the inner thread (7) of the legs (4, 5; 26, 27) is designed as a metric thread.

9. Element according to one of Claims 5 to 7, **characterized in that** the inner thread (7) of the legs (4, 5; 26,a b) is designed as a thread with a negative flank angle of the load-bearing flank.

10. Element according to one of Claims 5 to 7, **characterized in that** the inner thread (7) of the legs (4, 5; 26, 27) is designed as a buttress thread with a substantially horizontal angle of the load-bearing flank (29a).

11. Element according to one of Claims 5 to 7, **characterized in that** the inner thread (7) of the legs (4, 5; 26, 27) is designed as a flat thread with two horizontal flanks (29a, 29b).

12. Element according to one of Claims 5 to 11, **characterized in that** the outer thread (9) of the fastener element (8, 80) is designed with a thread type corresponding to the inner thread (7; 29) of the legs (4, 5; 26, 27).

13. Element according to one of Claims 5 to 12, **characterized in that** the fastener element (80) has a limit stop (81).

14. Element according to one of Claims 5 to 13, **characterized in that** the shaft (1) and the retaining element (2) are connected monoaxially.

15. Element according to one of Claims 5 to 13, **characterized in that** the shaft (1) and the retaining element (21; 21') are connected multiaxially.

16. Element according to Claim 15, **characterized in that** a pressure element (40) provided in the retaining element (21') is provided, on which the fastener element (8, 80) acts in order to fix the angle position of the shaft (1).

17. Element according to Claim 16, **characterized in that** the angle position of the shaft can be fixed via the fastener element (8, 80), and the rod via the inner screw (15).

## Revendications

1. Dispositif de fixation pour sécuriser un élément en forme de barre dans un élément de retenue (2 ; 21 ; 21') relié à une tige (1), pour utilisation dans la chirurgie de la colonne vertébrale ou des accidents, avec un élément de fixation (8 ; 80) ayant une forme sensiblement cylindrique creuse, avec un axe de rotation, un filetage extérieur (9) et un filetage intérieur (10),
**caractérisé par** une fente (14) s'étendant en direction axiale à travers l'élément de fixation.

2. Dispositif de fixation selon la revendication 1,
**caractérisé par** une vis intérieure (15) susceptible d'être vissée dans l'élément de fixation (8 ; 80).

3. Dispositif de fixation selon la revendication 1 ou 2, **caractérisé en ce que** le filetage intérieur (10) est réalisé sous la forme de filetage métrique.

4. Dispositif de fixation selon l'une des revendications 1 à 3, **caractérisé en ce que** le filetage extérieur (9) est réalisé sous la forme de filetage métrique ou de filetage à angle horizontal ou négatif du flanc de charge, ou sous forme de filetage plat avec deux flancs horizontaux.

5. Elément avec une tige (1) et un élément de retenue (2 ; 21 ; 21') lui étant relié, pour assurer la liaison à une barre (100), l'élément de retenue (2 ; 21 ; 21') présentant un évidement à section transversale en forme de U pour recevoir la barre, avec deux branches (4, 5 ; 26, 27) libres, à une extrémité (6 ; 28), et un filetage intérieur (7 ; 29) sur les branches libres; et avec un élément de fixation (8 ; 80) ayant un filetage extérieur (9) en au moins un tronçon de sa paroi extérieure, le filetage extérieur (9) coopérant avec le filetage intérieur (7 ; 29) des branches, et l'élément de fixation présentant un perçage central, **caractérisé en ce que** l'élément de fixation (8, 80) présente une fente (14) s'étendant sensiblement en direction axiale à travers sa paroi extérieure.

6. Élément selon la revendication 5, **caractérisé en ce que** l'élément de fixation (8 ; 80) présente un filetage intérieur (10) et une vis intérieure (15), pour vissage dans l'élément de fixation.

7. Élément selon la revendication 5 ou 6, **caractérisé en ce que** le filetage intérieur (10) de l'élément de fixation (8 ; 80) et le filetage extérieur (16), coopérant avec lui, de la vis intérieure (15) sont réalisés sous forme de filetage métrique.

8. Élément selon l'une des revendications 5 à 7, **caractérisé en ce que** le filetage intérieur (7) des branches (4, 5 ; 26, 27) est réalisé sous la forme de filetage métrique.

9. Élément selon l'une des revendications 5 à 7, **caractérisé en ce que** le filetage intérieur (7) des branches (4, 5 ; 26, a b) est réalisé sous la forme de filetage, avec un angle de flanc négatif du flanc de charge.

10. Élément selon l'une des revendications 5 à 7, **caractérisé en ce que** le filetage intérieur (7) des branches (4, 5 ; 26, 27) est réalisé sous la forme de filetage en dents de scie, avec un angle sensiblement horizontal du flanc de charge (29a).

11. Élément selon l'une des revendications 5 à 7, **caractérisé en ce que** le filetage intérieur (7) des branches (4, 5 ; 26, 27) est réalisé sous forme de filetage plat à deux flancs horizontaux (29a, 29b).

12. Élément selon l'une des revendications 5 à 11,
**caractérisé en ce que** le filetage extérieur (9) de l'élément de fixation (8. 80) est réalisé avec un type de filetage s'ajustant au filetage intérieur (7 ; 29) des branches (4, 5 ; 26, 27).

13. Élément selon l'une des revendications 5 à 12, **caractérisé en ce que** l'élément de fixation (80) présente une butée (81).

14. Élément selon l'une des revendications 5 à 13, **caractérisé en ce que** la tige (1) et l'élément de retenue (2) sont reliés de façon monoaxiale.

15. Élément selon l'une des revendications 5 à 13, **caractérisé en ce que** la tige (1) et l'élément de retenue (21 ; 21') sont reliés de façon polyaxiale.

16. Élément selon la revendication 15, **caractérisé en ce qu'**un élément de pressage (40) prévu dans l'élément de retenue (21') est prévu sur lequel l'élément de fixation (8, 80) agit pour la fixation de la position angulaire de la tige (1).

17. Élément selon la revendication 16, **caractérisé en ce que** la position angulaire de la tige est susceptible d'être fixée par l'intermédiaire de l'élément de fixation (8, 80). et la barre est susceptible d'être fixée par l'intermédiaire de la vis intérieure (15).
